Europäisches Patentamt

European Patent Office

Office européen des brevets

(11) Veröffentlichungsnummer: **0 162 403**
**A2**

(12) **EUROPÄISCHE PATENTANMELDUNG**

(21) Anmeldenummer: **85105894.1**

(22) Anmeldetag: **14.05.85**

(51) Int. Cl.⁴: **A 61 M 15/00**

(30) Priorität: **23.05.84 DE 3419148**

(43) Veröffentlichungstag der Anmeldung:
**27.11.85 Patentblatt 85/48**

(84) Benannte Vertragsstaaten:
**AT BE CH FR GB IT LI LU NL SE**

(71) Anmelder: **Klimt, Hans Ulrich**
**Rufenbergweg 14**
**D-7912 Weissenhorn-Bubenhausen(DE)**

(72) Erfinder: **Klimt, Hans Ulrich**
**Rufenbergweg 14**
**D-7912 Weissenhorn-Bubenhausen(DE)**

(74) Vertreter: **Vogel, Georg**
**Hermann-Essig-Strasse 35 Postfach 105**
**D-7141 Schwieberdingen(DE)**

(54) **Inhalationsgerät.**

(57) Die Erfindung betrifft ein Inhalationsgerät mit einem Behälter zur Aufnahme einer Inhalationsflüssigkeit, bei dem die Spitze eines elektromotorisch in Drehbewegungen versetzbaren Ansaugkegels in die Inhalationsflüssigkeit eintaucht und im Bereich seines oben liegenden Umfangsrandes eine Sprühzone erzeugt, bei dem ein Luft-Einlaß vorgesehen ist, der im Bereich der Sprühzone endet, und bei dem über einen Aerosol-Ausgang die mit der über den Luft-Einlaß zugeführten Luft vermischte Inhalationsflüssigkeit als Aerosol abgegeben wird. Die Vernebelung und die Vermischung wird nach der Erfindung mit einem einfachen, leichten Ansaugkegel dadurch verbessert, daß sich an den Umfangsrand des Ansaugkegels nach oben Flügel eines Radialgebläses anschließen, das mit dem Ansaugkegel verbunden und in Drehbewegungen versetzbar ist und daß der Luft-Einlaß in dem oder über dem Raum endet, der von den Flügeln des Radialgebläses umschlossen ist.

Fig. 1

Inhalationsgerät

Die Erfindung betrifft ein Inhalationsgerät mit einem Behälter zur Aufnahme einer Inhalationsflüssigkeit, bei dem die Spitze eines elektromotorisch in Drehbewegungen versetzbaren Ansaugkegels in die Inhalationsflüssigkeit eintaucht und im Bereich seines oben liegenden Umfangrandes eine Sprühzone erzeugt, bei dem ein Luft-Einlaß vorgesehen ist, der im Bereich der Sprühzone endet, und bei dem über einen Aerosol-Ausgang die mit der über den Luft-Einlaß zugeführten Luft vermischte Inhalationsflüssigkeit als Aerosol abgegeben wird.

Ein Inhalationsgerät dieser Art ist durch die DE-OS 32 42 128 bekannt. Der Ansaugkegel fördert bei seiner Drehbewegung die Inhalationsflüssigkeit auf seiner Außenfläche bis zum Umfangsrand hoch. Dort wird sie aufgrund der Zentrifugalkraft weggeschleudert, so daß sie vernebelt wird und eine Sprühzone entsteht. Als Luft-Einlaß sind mehrere Ansaugrohre vorgesehen, die parallel zur Antriebswelle des Ansaugkegels ausgerichtet und so um den Ansaugkegel gruppiert sind, daß ihre Öffnungen unterhalb des Umfangsrandes des Ansaugkegels und damit der Sprühzone angeordnet sind. Der Aerosol-Ausgang liegt über der Sprühzone, so daß die angesaugte Luft auf dem Weg zum Aerosol-Ausgang die Sprühzone passieren muß, wobei sie sich mit der vernebelten Inhalationsflüssigkeit vermischt.

Bei dem bekannten Inhalationsgerät läßt sich durch die unsymmetrische Anordnung von Ansaugkegel und Luft-Einlaß keine gleichmäßige Vermischung zwischen der angesaugten Luft und der vernebelten Inhalationsflüssigkeit erreichen. Außerdem ist die Vernebelung der Inhalationsflüssigkeit auch nicht fein genug und hängt sehr stark von der Saugwirkung am Aerosol-Ausgang ab.

Eine Verbesserung der Vernebelung der Inhalationsflüssigkeit läßt sich bei einem Inhalationsgerät nach dem DE-GM 83 28 261 erreichen. Bei diesem bekannten Inhalationsgerät ist vorgesehen, daß der hohle Ansaugkegel im Anschluß an seinen Umfangsrand mittels eines hohlzylinderförmigen Verwirbelungsteils verlängert ist, daß der Ansaugkegel mit einer stirnseitigen Kammer des Verwirbelungsteils in Verbindung steht, daß von der Kammer radial gerichtete Bohrungen ausgehen, daß die dem Ansaugkegel abgekehrte Stirnseite des Verwirbelungsteils eine zentrische Vertiefung aufweist, von der weitere radial gerichtete Bohrungen ausgehen, daß der hülsenförmige Luft-Einlaß konzentrisch zur Antriebswelle des Ansaugkegels angeordnet ist und in die Vertiefung des Verwirbelungsteils eingeführt ist und daß der Aerosol-Ausgang über dem zylindrischen Verwirbelungsteil liegt.

Bei diesem bekannten Inhalationsgerät wird die Inhalationsflüssigkeit auf der Außen- und der Innenwand des hohlen Ansaugkegels hochgefördert und an dem Umfangsrand bzw. durch die radialen Bohrungen des Verwirbelungsteils vernebelt. Dabei nimmt wohl die Menge der geförderten Inhalationgsflüssigkeit zu, aber der Grad der Vernebelung wird nicht verbessert. Mit der zentrischen Zufuhr der angesaugten Luft wird die Vermischung der vernebelten Inhalationsflüssigkeit mit der angesaugten Luft etwas verbessert. Dies bedingt aber einen voluminösen und komplizierten Ansaugkegel, der gerade bei der benötigten, sehr hohen Drehzahl von Nachteil ist.

Es ist Aufgabe der Erfindung, ein Inhalationsgerät der eingangs erwähnten Art zu schaffen, bei dem mit einem einfachen, leichten Ansaugkegel nicht nur der Grad der Vernebelung, sondern auch die Vermischung der vernebelten Inhalationsflüssigkeit mit der angesaugten Luft wesentlich verbessert wird.

Diese Aufgabe wird nach der Erfindung dadurch gelöst, daß sich an den Umfangsrand des Ansaugkegels nach oben Flügel eines Radialgebläses anschließen, das mit dem Ansaugkegel verbunden und in Drehbewegungen versetzbar ist und daß der Luft-Einlaß in dem oder über dem Raum endet, der von den Flügeln des Radialgebläses umschlossen ist.

Bei dieser Ausgestaltung des Ansaugkegels wird gerade im Bereich des Umfangrandes des Ansaugkegels, an dem die Vernebelung der geforderten Inhalationsflüssigkeit erfolgt, durch das Radialgebläse ein Luftstrom erzeugt, der symmetrisch und mit Druck der vernebelten Inhalationsflüssigkeit überlagert und mit dieser vermischt wird. Dabei ist diese Vermischung unabhängig von der Saugwirkung am Aerosol-Ausgang. Es wird eine ausgezeichnete Vermischung von angesaugter Luft und geförderter Inhalationsflüssigkeit erreicht und auch der Grad der Verneblung wird verbessert, da die unter Druck zugeführte Luft im Bereich des Umfangsrandes des Ansaugkegels die Vernebelung, d.h. die Bildung kleinerer Tröpfchen der Inhalationsflüssigkeit, begünstigt. Der Ansaugkegel bleibt dennoch klein und einfach.

Die Bildung des Radialgebläses wird nach einer einfachen Art dadurch vorgenommen, daß die Flügel durch Längsschlitze an einem Hohlzylinder abgeteilt sind, der einstückig mit dem Umfangsrand des Ansaugkegels verbunden ist, wobei der Hohlzylinder radial und in Richtung zur Spitze am Umfangsrand vorsteht und die Längsschlitze sich bis zum Umfangsrand des Ansaugkegels erstrecken. Durch diesen Übergang zwischen dem Ansaugkegel und den Flügeln des Radialgebläses wird die Vernebelung verbessert, da die Druckluft des Radialgebläses im Bereich des Umfangsrandes des Ansaugkegels mit der geförderten Inhalationsflüssigkeit zusammengeführt wird. Dabei ist zudem vorgesehen, daß die Innenseiten der Flügel gegenüber dem Umfangsrand des Ansaugkegels geringfügig zur Mitte des Ansaugkegels hin versetzt sind, um möglichst wenig Material für die Flügel des Radialgebläses aufwenden zu müssen.

Der Vernebelungsgrad und der Vermischungsgrad werden nach einer weiteren Ausgestaltung dadurch noch verbessert, daß die dem Umfangsrand des Ansaugkegels zugekehrten Stirnseiten der Flügel vom Umfangsrand des Ansaugkegels ausgehen und sich in Richtung zur Spitze desselben mit kontinuierlich größer werdendem Abstand zum Ansaugkegel von diesem abstehen.

Ist nach einer Ausgestaltung vorgesehen, daß der Ansaugkegel mit den Flügeln als Kunststoff-Spritzgußteil hergestellt ist, das drehfest mit einer Antriebswelle eines Elektromotors verbunden ist, dann kann der Ansaugkegel dennoch als kostengünstiges Teil hergestellt werden.

Eine symmetrische Zufuhr der durch das Radialgehäuse angesaugten Luft wird nach einer Ausgestaltung dadurch erreicht, daß die Antriebswelle zentrisch zum Umfangsrand mit dem

Ansaugkegel verbunden ist und daß die Antriebswelle hohl ausgebildet ist und als Luft-Einlaß im Bereich zwischen den Flügeln des Radialgebläses mit Durchbrüchen versehen ist.

Die Zufuhr der angesaugten Luft zum Radialgebläse kann jedoch auch so erfolgen, daß als Luft-Einlaß ein Rohrabschnitt in dem Gehäuseteil festgelegt ist, das den Raum über den Flügeln des Radialgebläses begrenzt. Diese Zufuhr ist ausreichend, da das Radialgebläse selbst schon eine große Saugwirkung ausübt. Bei dieser Ausgestaltung bereitet die Lagerung der Antriebswelle des Ansaugkegels keine Schwierigkeiten mehr.

Nach einer Ausgestaltung ist vorgesehen, daß der Aerosol-Ausgang im Bereich der oberen Stirnseiten der Flügel des Radialgebläses radial gerichtet abgeht, um den Abzug des Aerosols über der Sprüh- und Mischzone zu haben.

Die Menge der geförderten Inhalationsflüssigkeit kann nach einer Ausgestaltung dadurch erhöht werden, daß der Ansaugkegel als Hohlkörper ausgebildet ist, der im Bereich der Spitze eine Öffnung aufweist.

Um einen ruhigen Lauf des mit hoher Drehzahl angetriebenen Ansaugkegels zu erhalten, sieht eine weitere Ausgestaltung vor, daß die Flügel in gleichmäßigen Abständen über den Umfangsrand des Ansaugkegels verteilt sind. Die Herstellung des Ansaugkegels mit den Flügeln des Radialgebläses wird nach einer Ausgestaltung dadurch vereinfacht, daß die Flügel an dem Hohlzylinder durch Längsschlitze abgeteilt sind, die in Längsrichtung der Flügel und über die gesamte Dicke der Flügel eine gleichbleibende Breite aufweisen.

Eine vorteilhafte Ausgestaltung ist dadurch gekennzeichnet, daß die Flügel und die Längsschlitze etwa gleiche Breite aufweisen.

Die Erfindung wird anhand eines in den Zeichnungen dargestellten Ausführungsbeispiels näher erläutert. Es zeigt:

Fig. 1 im Längsschnitt den Teil des Inhalationsgerätes mit dem Ansaugkegel und dem Radialgebläse und

Fig. 2 einen Querschnitt entlang der Linie II-II der Fig. 1 mit der Anordnung der Flügel an dem Ansaugkegel.

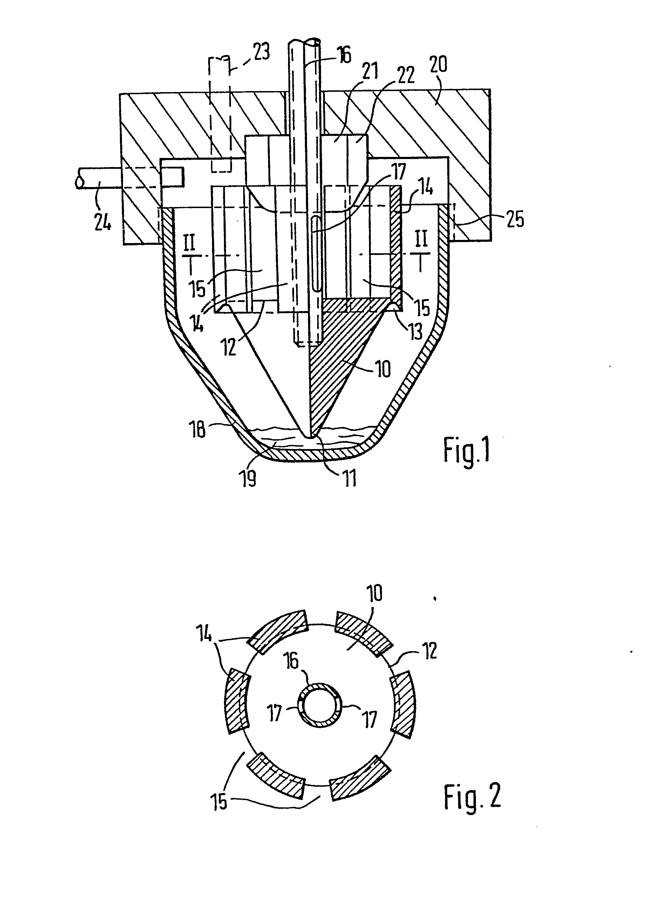

Die Inhalationsflüssigkeit wird bei dem Inhalationsgerät in den topfartigen Behälter 18 eingefüllt, der vorzugsweise aus durchsichtigem Material besteht. Der Behälter 18 wird in bekannter Weise an dem Gehäuseteil 20 befestigt, wie die als Bajonett- oder Schraubverbindung ausgebildete Verbindung 25 andeutet. In dem Gehäuseteil 20 ist die Antriebswelle 16 des Elektromotors drehbar gelagert, wie das aus den Teilen 21 und 22 bestehende Lager zeigt. Das untere Ende der hohlen Antriebswelle 16 ist in dem massiven Ansaugkegel 10 drehfest befestigt, so daß dieser in entsprechende Drehbewegungen versetzt werden kann. Der obere Umfangsrand 12 des Ansaugkegels 10 geht in das aus den Flügeln 14 bestehende Radialgebläse über. Die Flügel 14 sind einstückig an dem Umfangsrand 12 des Ansaugkegels 10 angeformt. Dabei sind die Innenseiten der Flügel 14 gegenüber dem Umfangsrand 12 des Ansaugkegels 10 geringfügig zur Mitte des Ansaugkegels 10 hin versetzt. Die Flügel 14 sind durch die Längsschlitze 15 abgeteilt. Wie der Querschnitt nach Fig. 2 zeigt, sind die Flügel 14 Teil eines Hohlzylinders, der durch die Längsschlitze 15 gleichmäßig unterteilt ist. Dabei bleiben die Flügel 14 stehen. Die Längsschlitze 15 haben in Längsrichtung der Flügel 14 und in der Dicke der Flügel 14 eine gleichbleibende Breite, die die einstückige Herstellung des Ansaugkegels 10 mit den Flügeln 14 erleichtert. Die Längsschlitze 15 erstrecken sich bis zum Umfangsrand 12 des Ansaugkegels 10, so daß die durch das Radialgebläse angesaugte und radial nach außen gedrückte Luft unmittelbar im Bereich des Umfangsrandes 12, an dem auch die Vernebelung der am Ansaugkegel 10 hochgeförderten Inhalationsflüssigkeit erfolgt, der vernebelten Inhalationsflüssigkeit beigemischt wird.

Ist die Antriebswelle 16 hohl ausgebildet und hat sie in dem von den Flügeln 14 umschlossenen Raum die Durchbrüche 17, dann wird die angesaugte Luft symmetrisch zugeführt und von dem Radialgebläse der entstehende Sprühzone vernebelter Inhalationsflüssigkeit symmetrisch überlagert.

Da das Radialgebläse eine ausreichende Ansaugwirkung hat, genügt es auch, den Luft-Einlaß über dem von den Flügeln 14 des Radialgebläses umschlossenen Raum enden zu lassen, wie der im Gehäuseteil 20 festgelegte Rohrabschnitt 23 andeutet. Die Drehlagerung der Antriebswelle 16 wird dadurch einfacher, da darüber die angesaugte Luft nicht mehr zugeführt werden muß.

Die unteren Stirnseiten 13 der Flügel 14 stehen am Umfangsrand 12 in Richtung zur Spitze 11 des Ansaugkegels 10 hin vor, und zwar vergrößert sich der Abstand zum Ansaugkegel 10 zur Spitze 11 hin kontinuierlich. Damit wird die Bildung der Sprühzone verbessert.

Der Aerosol-Auslaß 24 geht als Rohrabschnitt im Gehäuseteil 20 radial vom Ansaugkegel 10 ab und geht etwas von dem Bereich der oberen Stirnseiten der Flügel 14 aus, damit er über der Sprühzone liegt.

Der Ansaugkegel 10 kann auch hohl ausgebildet werden, wobei er an der Spitze 11 eine Öffnung hat. Auf diese Weise läßt sich ein zusätzlicher Förderstrom an Inhalationsflüssigkeit auf der Innenwandung des Ansaugkegels 10 schaffen, der ebenfalls im Bereich des Umfangsrandes 12 endet und vernebelt wird. Der Ansaugkegel 10 trägt innen nur Stege und eine Hülse, um die Antriebswelle 16 drehfest aufnehmen zu können.

Hans Ulrich Klimt
Rufenbergweg 14

7912 Weißenhorn-
 Bubenhausen

- 1 -

<u>A n s p r ü c h e</u>

1. Inhalationsgerät mit einem Behälter zur Aufnahme einer
   Inhalationsflüssigkeit, bei dem die Spitze eines elektromotorisch in Drehbewegungen versetzbaren Ansaugkegels in die Inhalationsflüssigkeit eintaucht und im
   Bereich seines oben liegenden Umfangrandes eine Sprühzone erzeugt, bei dem ein Luft-Einlaß vorgesehen ist,
   der im Bereich der Sprühzone endet, und bei dem über
   einen Aerosol-Ausgang die mit der über den Luft-Einlaß
   zugeführten Luft vermischte Inhalationsflüssigkeit als
   Aerosol abgegeben wird,
   dadurch gekennzeichnet,
   daß sich an den Umfangsrand (12) des Ansaugkegels (10)
   nach oben Flügel (14) eines Radialgebläses anschließen,
   das mit dem Ansaugkegel (10) verbunden und in
   Drehbewegungen versetzbar ist und
   daß der Luft-Einlaß in dem oder über dem Raum endet,
   der von den Flügeln (14) des Radialgebläses umschlossen
   ist.

2. Inhalationsgerät nach Anspruch 1,
   dadurch gekennzeichnet,
   daß die Flügel (14) durch Längsschlitze (15) an einem
   Hohlzylinder abgeteilt sind, der einstückig mit dem
   Umfangsrand (12) des Ansaugkegels (10) verbunden ist,
   wobei der Hohlzylinder radial und in Richtung zur
   Spitze (11) am Umfangsrand (12) vorsteht und die Längsschlitze (15) sich bis zum Umfangsrand (12) des Ansaugkegels (10) erstrecken.

3. Inhalationsgerät nach Anspruch 2,
   dadurch gekennzeichnet,
   daß die Innenseiten der Flügel (14) gegenüber dem
   Umfangsrand (12) des Ansaugkegels (10) geringfügig zur
   Mitte des Ansaugkegels (10) hin versetzt sind.

4. Inhalationsgerät nach Anspruch 2 oder 3,
   dadurch gekennzeichnet,
   daß die dem Umfangsrand (12) des Ansaugkegels (10)
   zugekehrten Stirnseiten (13) der Flügel (14) vom Umfangsrand (12) des Ansaugkegels (10) ausgehen und sich
   in Richtung zur Spitze (11) desselben mit kontinuierlich größer werdendem Abstand zum Ansaugkegel (10) von
   diesem abstehen.

5. Inhalationsgerät nach einem der Ansprüche 1 bis 4,
   dadurch gekennzeichnet,
   daß der Ansaugkegel (10) mit den Flügeln (14) als
   Kunststoff-Spritzgußteil hergestellt ist, das drehfest
   mit einer Antriebswelle (16) eines Elektromotors verbunden ist.

6.  Inhalationsgerät nach Anspruch 5, .
    dadurch gekennzeichnet,
    daß die Antriebswelle (16) zentrisch zum Umfangsrand
    (12) mit dem Ansaugkegel (10) verbunden ist und
    daß die Antriebswelle (16) hohl ausgebildet ist und als
    Luft-Einlaß im Bereich zwischen den Flügeln (14) des
    Radialgebläses mit Durchbrüchen (17) versehen ist.

7.  Inhalationsgerät nach einem der Ansprüche 1 bis 5,
    dadurch gekennzeichnet,
    daß als Luft-Einlaß ein Rohrabschnitt (23) in dem
    Gehäuseteil (20) festgelegt ist, das den Raum über den
    Flügeln (14) des Radialgebläses begrenzt.

8.  Inhalationsgerät nach einem der Ansprüche 1 bis 7,
    dadurch gekennzeichnet,
    daß der Aerosol-Ausgang (24) im Bereich der oberen
    Stirnseiten der Flügel (14) des Radialgebläses radial
    gerichtet abgeht.

9.  Inhalationsgerät nach einem der Ansprüche 1 bis 8,
    dadurch gekennzeichnet,
    daß der Ansaugkegel (10) als Hohlkörper ausgebildet
    ist, der im Bereich der Spitze (11) eine Öffnung auf-
    weist.

10. Inhalationsgerät nach einem der Ansprüche 1 bis 9,
    dadurch gekennzeichnet,
    daß die Flügel (14) in gleichmäßigen Abständen über den
    Umfangsrand (12) des Ansaugkegels (10) verteilt sind.

11. Inhalationsgerät nach einem der Ansprüche 1 bis 10,
    dadurch gekennzeichnet,
    daß die Flügel (14) an dem Hohlzylinder durch Längsschlitze (15) abgeteilt sind, die in Längsrichtung der
    Flügel (14) und über die gesamte Dicke der Flügel (14)
    eine gleichbleibende Breite aufweisen.

12. Inhalationsgerät nach Anspruch 11,
    dadurch gekennzeichnet,
    daß die Flügel (14) und die Längsschlitze (15) etwa
    gleiche Breite aufweisen.

Fig.1

Fig.2